# EUROPEAN PATENT APPLICATION

(11) **EP 0 897 730 A2**
(43) Date of publication of application: **24.02.1999**
(21) Application number: 98306397.5
(22) Date of filing: 11.08.1998
(51) Int. Cl.: A61M 29/02

(54) **Retainer for a stent-carrying balloon catheter**

(30) Priority: 11.08.1997 US 909398
(71) Applicant: Advanced Cardiovascular Systems, Inc., Santa Clara, California 95054 (US)
(72) Inventor: Wantink, Kenneth L., Temecula, California 92591 (US)
(74) Representative: McLeish, Nicholas Alistair Maxwell

(57) **Abstract**

A retainer for a stent-carrying balloon catheter, which prevents rolling up of the distal end of a protective sheath during advancement of the catheter in a vessel, such as a coronary artery. The sheath distal end adjacently covers a distal end of the balloon catheter without being secured thereto. The retainer extends over and encloses the expandable sheath, and is secured to the distal end of the balloon catheter, without securing the sheath distal end to the balloon catheter.

## Description

### BACKGROUND OF THE INVENTION

### Field of the Invention

This invention relates to a protective retainer for protecting the distal end of a balloon catheter assembly, for example of the kind used in delivering an intravascular stent.

### Description of the Related Art

In a typical percutaneous transluminal coronary angioplasty (PTCA) for compressing lesion plaque against the artery wall to dilate an arterial lumen, a guiding catheter is introduced percutaneously into the cardiovascular system of a patient through the brachial or femoral artery and is advanced through the vasculature until the distal end is in the ostium. A guide wire and a dilatation catheter having a ballon on the distal end are introduced through the guiding catheter with the guide wire sliding within the dilatation catheter. The guide wire first is advanced out of the guiding catheter into the coronary vasculature of the patient, and the dilatation catheter is advanced over the previously positioned guide wire until the dilatation balloon is properly oriented across the lesion. Once in position across the lesion, a flexible, expandable, pre-formed balloon is inflated to a pre-determined size at relatively high pressures to radially compress the atherosclerotic plaque of the lesion against the inside of the artery wall and thereby to dilate the lumen of the artery. The balloon then is deflated to a small profile, so that the dilatation catheter can be withdrawn from the vasculature and the blood flow resumed through the dilated artery. While this procedure is typical, it is not the only method used in angioplasty.

In angioplasty procedures of the kind described above, a restenosis of the artery often occurs, which may require another angioplasty procedure, a surgical bypass operation, or some other method of repairing or strengthening the area. To reduce the chance of the development of restenosis and to strengthen the area, a physician can implant an intravascular prosthesis, typically called a stent, for maintaining vascular patency. A stent is a device that is used to hold tissue in place or to provide a support for a vessel to hold it open so that blood flows freely. A variety of devices are known in the art for use as stents, including expandable tubular members, in a variety of patterns, that are capable of being crimped onto a balloon catheter, and then being expanded upon being positioned intraluminally on the balloon catheter, and which can retain the post-deployment form. Typically, the stent is loaded and crimped onto the balloon portion of the catheter, and then is advanced to a location inside the artery at the lesion. The stent subsequently is expanded to a larger diameter, by the balloon portion of the catheter, to implant the stent in the artery at the lesion. Typical stents and stent delivery systems are more fully disclosed in U.S. Patent No. 5,514,154 (Lau et al.) and U.S. Patent No. 5,507,768 (Lau et al.)

When an over-the-wire catheter or a rapid exchange-type catheter is used to deploy a stent, in order to protect the balloon portion of the catheter from the metal stent, and to effect a more secure attachment interface, a protective tubular sheath may be placed over the balloon portion, and the stent may be crimped onto the sheath. In some prior art devices, the sheath may be attached to the proximal and distal ends of the catheter. However, if the balloon ruptures, the fluid used to inflate the balloon can become trapped in the sheath, such that the sheath may assume an inflated state and consequently may interfere with withdrawal of the catheter from the patient. If the sheath is open at the distal end, so as to prevent the inflation fluid from becoming trapped therein, the sheath undesirably may roll up when the catheter is advanced into a vessel or artery.

### SUMMARY OF THE INVENTION

Through its various embodiments, the present invention attempts to solve the problems associated with prior art balloon catheters which have been used to delivery intravascular stents.

Preferred embodiments are directed to a retainer for an over-the-wire or rapid exchange ("RX") stent-carrying balloon catheter, wherein the retainer prevents the protective sheath from rolling up at the distal end thereof when the catheter is advanced through an artery or other blood vessel. The distal end of the sheath adjacently covers the distal end of the balloon catheter but is not attached to the balloon catheter distal end.

In one embodiment of the present invention, the retainer is tubular, extends over and encloses the distal end of the protective sheath, and is secured to the distal end of the catheter, to prevent the sheath from rolling up during insertion of the catheter in the artery of the patient. The sheath distal end is not secured to the balloon catheter, so as to prevent trapping of inflation fluid therein if the balloon ruptures. The retainer is formed from an elastic, flexible material and can be attached to the catheter distal end by, for example, laser welding. The tapered distal tip of the retainer is soft and atraumatic to facilitate advancement through the artery or other vessel.

These and other advantages of the invention will become more apparent from the following detailed description thereof when taken in conjunction with the accompanying drawings.

### BRIEF DESCRIPTION OF THE DRAWINGS

FIGURE 1 is an axial cross-section of the distal portion of a stent delivery catheter having an elastic membrane covering the balloon portion of the catheter and catheter shaft, where the balloon is in the deflated state.

FIG. 2 is an axial cross-section of the distal end of the stent delivery catheter of FIG. 1 in which the balloon and elastic membrane are inflated.

FIG. 3 is a cross-sectional view of a preferred embodiment of the invention, depicting the retainer covering the distal end of the protective sheath and the distal end of the stent delivery catheter.

### DETAILED DESCRIPTION OF THE PREFERRED EMBODIMENTS

After an angioplasty procedure, whether performed as a PTCA procedure, through use of atherectomy devices, or otherwise accomplished by removing or reducing a stenotic lesion, it is desirable to deliver and implant an intravascular stent. It has been determined that intravascular stents not only support a lumen, but also may reduce the likelihood of the development of restenosis therein.

A stent delivery catheter, having a balloon at its distal and, typically is used to deliver and implant the stent within the body lumen, such as a coronary artery. In order to protect the balloon and to more securely attach the stent to the catheter prior to implanting in the artery, a protective tubular sheath, which typically is an elastic membrane, can be positioned over the balloon portion of the catheter. In order to prevent the distal end of the protective sheath from curling up during delivery of the stent, a retainer, formed from a substantially elastic material, is placed over the distal end of the protective sheath.

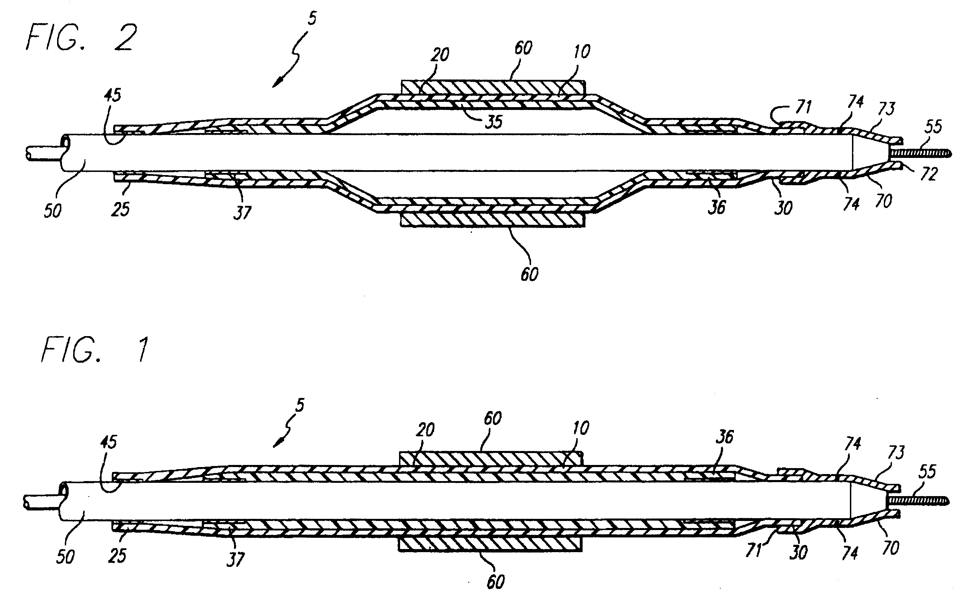

As shown in FIGS. 1 and 2, the catheter 5 has attached at its distal end an elastic, resilient, membrane layer 10, that forms a tubular sheath 20 over the balloon 35. The membrane layer 10 may be formed of any suitable material that is elastic and resilient. The material from which the membrane layer is formed preferably is one that has a high degree of linearity (non-plasticity) for a wide range of stress and strain values. Commercially available tubing such as C-FLEX tubing can be used. C-FLEX tubing may be obtained from Concept Polymer Technologies of Largo, Florida, U.S.A. In addition, the material should have good tear strength to prevent fracturing or splitting when it is stretched. Suitable materials include silicones, latexes, urethanes, polysiloxane modified styrene-ethylene/butylene-styrene block copolymers (SEBS) and the families of materials associated therewith.

As is shown in FIG. 2, the membrane layer 10, in the form of a tubular sheath 20 having a proximal end 25 and a distal end 30, surrounds the outside of the balloon 35 of a balloon catheter 5. The tubular sheath 20 is affixed by adhesive to the catheter 5 at a point 45 on the catheter shaft or outer member 50, with the point 45 lying at the proximal end 25 of the tubular sheath and lying outside the balloon 35 of the catheter, which has a proximal portion 37. The balloon is affixed to the catheter shaft at the balloon proximal portion 37, such as by adhesive or laser welding.

It can be seen how the sheath 20 entirely overlies and covers the underlying balloon 35 of the catheter. As is known in the art, the balloon 35 of the catheter 5 either is bonded to the outer member 50 or is made one-piece with the outer member. The catheter balloon can be inflated by an inflation fluid from an inflating port (not shown) which extends from a lumen contained in the catheter shaft, or by other means, depending on the design of the catheter, such as by fluid communicated from a passageway formed between the outside of the catheter shaft and the membrane forming the balloon. The details and mechanics of balloon inflation vary according to the design of the catheter, and are known in the art.

In the preferred embodiment, the balloon 35 has a distal end 36 and a proximal and 37, both of which are completely covered by the tubular sheath 20. As can be seen from the drawings, the tubular sheath 20 is affixed to the catheter outer member 50 at a point 45 just proximal to the proximal end 37 of the balloon 35, so that the tubular sheath 20 overlies the entire balloon portion of the catheter.

As is shown in FIGS. 1 and 2, the catheter 5 has an axially-extending outer member 50, which passes over a guide wire 55. The tubular sheath 20 is attached to the catheter outer member 50 at a point 45 proximal to the balloon 35. The distal end 30 of the tubular sheath 20 is not attached to the balloon 35 or to the catheter outer member 50 because, in the event of a rupture, the sheath 20 will be fastened at the proximal end, upstream from the location of the tear, and thus the sheath will be prevented from curling or bunching when the catheter is withdrawn. The distal end of the tubular sheath is not attached and remains open. If the balloon ruptures when it is inflated, any inflation fluid released will pass out through the open distal end of the tubular sheath 20.

Although the drawing figures depict an over-the-wire balloon catheter, embodiments of the invention may be used with any other type of catheter, including rapid-exchange balloon catheters.

Further, the tubular sheath 20 may be attached to the catheter not only by adhesive or laser bonding, but also by mechanical means. For example, the sheath may be attached by mechanical means such as fasteners that are made of radiopaque material, such as radiopaque collars around the proximal end of the sheath, to better define the outer limits of the sheath for a fluoroscopic viewing.

After securing the elastic tubular sheath 20 to the catheter 5, a stent 60 is positioned over the sheath. The stent typically is at least 15 mm long, while the tubular sheath 20 typically is about 30 mm long, the sheath always being longer than the stent and the balloon.

The stent 60 is positioned over the sheath 20 and the balloon 35 of the catheter 5 und is crimped tightly onto the tubular sheath 20 either by hand or with a tool, such as crimping pliers. The tubular sheath provides a cushion or substrate onto which the stent is crimped, to further help secure the stent onto the sheath and therefore onto the balloon portion of the catheter. The elastic membrane may be made of material with a high coefficient of friction to help the stent tightly grip the balloon.

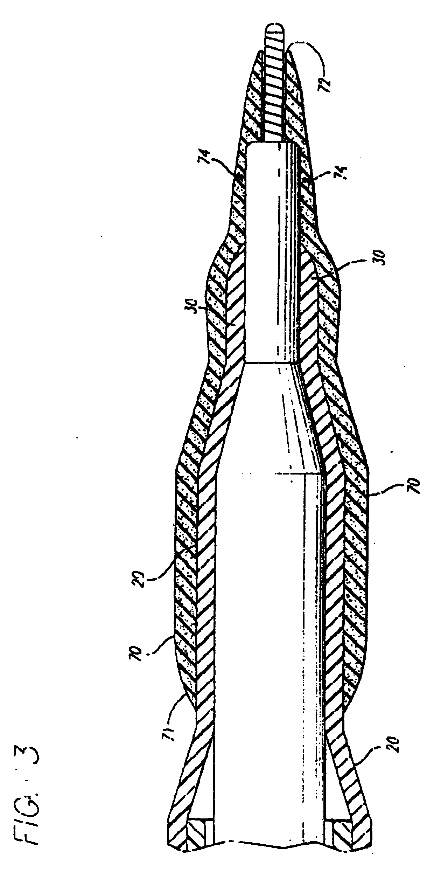

Turning to FIGS. 1-3, and particularly FIG. 3, a retainer 70 having a proximal end 71 and a distal end 72 is positioned over the distal end 30 of the tubular sheath 20 As described, the distal end 30 of the tubular sheath 20 is not attached to the catheter 5, the outer member 50 of the catheter 5, or the balloon 35. In other words, the distal end 30 of the tubular sheath 20 is intended to remain unattached in the unlikely event of a balloon rupture, so that balloon inflation fluid can pass freely into the patient's vessel rather than causing the tubular sheath to expand, which may impede removal of the catheter from the patient.

The retainer 70 has a tapered or necked soft tip 73 which is somewhat flexible and atraumatic so that the distal end 72 of the retainer 70 does not injure the patient's vessel wall during the delivery of the stent. The retainer 70 can be formed from a highly flexible and elastic material such as that manufactured under the trademark "HYTREL", by the E.I. Du Pont de Nemours Company. As more clearly depicted in FIG. 3, the proximal end 71 of the retainer 70 completely surrounds the distal end 30 of the tubular sheath 20, and due to its elastic properties, the retainer will tightly hold the distal end 30 on the catheter 5.

In order to secure the retainer 70 to the catheter, the retainer 70 is attached to the catheter 5 by a laser bond 74. It will be understood by those skilled in the art that other forms of attaching the retainer 70 to the catheter 5 are available, such as adhesives, heat sealing, and the like.

During delivery of the stent through the patient's vascular system, especially the coronary arteries, there will be tortuous passageways requiring the catheter 5 to be moved axially back and forth as the catheter is delivered over the guide wire 55. The retainer 70 provides the dual purpose of an atraumatic tip 73 which protects the vessel wall from injury, and prevents the distal end 30 of the tubular sheath 20 from curling up during the back and forth movement.

The retainer 70 can overlap the distal end 30 of the tubular sheath 20 by between 2 mm and 20 mm. The range of overlap is representative only, and can vary widely depending upon the application and the particular catheter assembly being used. The distal end 72 of the retainer 70 extends beyond the distal end of the catheter 5 a distance that insures that the tip 73 be atraumatic, yet will not interfere with the sliding movement of the catheter 5 over the guide wire 55 (not shown in FIG. 3).

The retainer 70 is made from a relatively thin material, to insure that the catheter assembly maintains a low profile during delivery. Thus, the retainer 70 should be in the range of about 0.025 to 0.076 mm (.0010 to .0030 inch) thick, and preferably about 0.038 mm (.0015 inch) thick. As will be understood by those skilled in the art, the objective is to maintain low profile delivery, and the thickness of the retainer may vary widely depending upon the catheter delivery system used as well as the application. For example, for stent delivery and implantation in a saphenous vein, where the diameter of the vessel is relatively large, a large delivery diameter and a retainer having a thicker wall section is acceptable even though smaller dimensions would be needed for deploying a stent in a coronary artery, where low profile diameters are essential.

The balloon catheter then is advanced over the guide wire 55 and is positioned in the patient's vasculature, so that the stent is adjacent to the portion of the vessel where treatment is to take place. The balloon is inflated along with the elastic tubular sheath, but not the retainer, to expand the stent to an enlarged diameter. During expansion, the sheath provides benefits such as protecting the balloon from rupture, preventing pinhole effects, preventing distortion of the stent into a "dog bone" shape, promoting even expansion of the stent, and discouraging the stent from moving axially along the catheter. When the stent has reached the desired diameter, the balloon is deflated. During deflation the tubular sheath reduces deflation time, allows the balloon to collapse in a uniform manner and prevents the balloon from assuming a "pancake" shape.

While in the preferred embodiment the balloon delivery catheter is the same or is similar to that used in therapeutic coronary angioplasty, it will be appreciated by those skilled in the art that modifications may be made to the present invention to allow the present invention to be used to implant any type of prosthesis. The present invention is not limited to stents and balloon delivery catheters that are deployed in a patient's vasculature, but has wide applications to delivering and implanting any type of graft, prosthesis, liner or similar structure. Further, the stent may be delivered by the balloon delivery catheter not only into coronary arteries, but into any body lumen, such as saphenous veins, peripheral veins and arteries, and other body lumens. Other modifications can be made to the present invention by those skilled in the art without departing from the scope thereof.

## Claims

1. A device for protecting the distal end (30) of a sheath (20) and the distal end of a balloon catheter (5) of the kind used to deliver and implant a stent (60), comprising:
a retainer (70) for enclosing the distal end of the sheath without securing the distal end of the sheath to the balloon catheter; and
means (74) for attaching the retainer to the distal end of the balloon catheter.

2. The device of claim 1, wherein the retainer (70) is generally tubular in shape.

3. The device of claim 1, wherein the retainer (70) includes a tip portion (73) which extends beyond the distal end of the balloon catheter.

4. The device of claim 1, wherein the attaching means (74) comprise a laser weld.

5. The device of claim 1, wherein the retainer (70) is comprised of material manufactured under the trademark "HYTREL" by the E.I. du Pont DeNemours Company.

6. The device of claim 1, wherein the retainer (70) is formed from an elastic material.

7. The device of claim 3, wherein the tip portion (73) comprises a tapered soft tip.

8. A method of preventing rolling up of a sheath (20) adapted to adjacently cover the distal end of a balloon catheter (5) of the kind used for delivering and implanting a stent (60), wherein a distal end (30) of the sheath is not secured to the balloon catheter, the method comprising:
positioning a retainer (70) for enclosing the distal end of the sheath so as to enclose the distal end of the sheath without securing the distal end of the sheath to the balloon catheter; and
securing the retainer to the distal end of the balloon catheter.

9. The method of claim 8, wherein the step of securing the retainer (70) to the distal end of the sheath comprises laser bonding (74).

10. An assembly for preventing rolling up of the distal end (30) of a sheath (20) adjacently covering a balloon portion (35) of a balloon catheter (5), without securing the distal end of the sheath to the balloon catheter, comprising:
a balloon catheter (5) having a proximal end and a distal end and a balloon portion (35) near the distal end;
a tubular sheath (20) having a distal end (30) and a proximal end (25) and positioned so as to adjacently cover a portion of the balloon catheter without being secured to a distal end of the balloon catheter; and
a retainer (70) secured to the distal end of the balloon catheter and enclosing the sheath so as to prevent rolling up of the sheath distal end (30) without securing the sheath distal and to the balloon catheter.

11. The assembly of claim 10, wherein the retainer (70) is secured to the distal end of the balloon catheter by laser bonding (74).
